Europäisches Patentamt

European Patent Office

Office européen des brevets

(19)

(11) **EP 0 864 557 B1**

(12) **FASCICULE DE BREVET EUROPEEN**

(45) Date de publication et mention
de la délivrance du brevet:
**30.01.2002 Bulletin 2002/05**

(51) Int Cl.⁷: **C07C 51/367**, C07C 59/52

(21) Numéro de dépôt: **98400490.3**

(22) Date de dépôt: **02.03.1998**

(54) **Procédé industriel de préparation en continu de l'orthohydroxymandélate de sodium**

Industrielles Verfahren zur kontinuierlichen Herstellung von Natriumorthohydroxamandelat

Industrial process for the continuous production of sodium orthohydroxymandelate

(84) Etats contractants désignés:
**AT BE CH DE ES FR GB IT LI LU NL SE**

(30) Priorité: **11.03.1997 FR 9702847**

(43) Date de publication de la demande:
**16.09.1998 Bulletin 1998/38**

(73) Titulaire: **Clariant (France)**
**92800 Puteaux (FR)**

(72) Inventeur: **Sidot, Christian**
**60200 Compiegne (FR)**

(74) Mandataire: **Rinuy, Santarelli**
**14, avenue de la Grande Armée, B.P. 237**
**75822 Paris Cédex 17 (FR)**

(56) Documents cités:
**EP-A- 0 556 084**      **FR-A- 2 495 137**

## Description

La présente invention concerne un procédé industriel de préparation de l'orthohydroxymandélate de sodium en continu, par condensation à chaud de l'acide glyoxylique avec le phénol en présence d'une amine tertiaire et de quantités catalytiques de cations métalliques trivalents.

L'orthohydroxymandélate de sodium peut être utilisé pour la fabrication discontinue de l'acide orthohydroxymandélique comme décrit dans le FR-A-2.687.143. Il est un intermédiaire précieux pour accéder soit à l'acide orthohydroxyphénylacétique utilisé pour l'obtention de molécules présentant d'intéressantes propriétés phytosanitaires, soit pour l'obtention de molécules de type acide éthylènediamine N,N'-bis(2-hydroxyphénylacétique) ou EDDHA pouvant former des complexes, notamment avec le fer ou le manganèse, soit pour l'obtention de nouveaux fongicides.

Il est connu que l'acide glyoxylique se condense avec le phénol en milieu aqueux alcalin pour conduire à un mélange d'acides ortho et parahydroxymandéliques, ainsi qu'aux acides hydroxybenzènediglycoliques substitués en -2,4 et en -2,6.

Il est également connu qu'en milieu aqueux alcalin, l'acide glyoxylique se dismute selon Cannizzaro en acides oxalique et glycolique. FR-A-2.440.350, décrit qu'en faisant réagir de l'acide glyoxylique avec du phénol pendant quelques minutes, à température élevée, dans un milieu aqueux alcalin, il était possible de minimiser la réaction de Cannizzaro et d'exalter la réaction de condensation et ainsi, de pouvoir atteindre un rendement de 70 à 85 % en acide parahydroxymandélique.

Par ailleurs, on sait préparer de l'acide parahydroxymandélique avec une bonne sélectivité, en milieu quasi anhydre, de manière discontinue, en faisant réagir de l'acide glyoxylique avec du phénol, en présence d'un excès d'amine tertiaire convenable tel que la tributylamine (comme décrit dans FR-A-2.638.740).

A.J. Hoefnagel et al, Rec. Trav. Chem., <u>107</u>, 242-7 (1988), ont montré que la condensation de l'acide glyoxylique avec du phénol pouvait être catalysée par certains ions métalliques et, qu'en opérant en milieu aqueux dilué, à pH = 5, à 100°C, en présence de cations métalliques trivalents tels que l'aluminium, le chrome, le fer, il était possible d'obtenir des sélectivités élevées en position ortho. Toutefois, en raison d'une part, d'une dilution importante qui fournit une faible productivité et d'autre part, de l'obtention d'un taux élevé en produits disubstitués qui conduit à des mélanges complexes dont il est difficile ensuite d'isoler l'acide orthohydroxymandélique cherché, ce procédé n'est pas industriellement économique.

WO-A-94.14746 décrit une méthode de séparation des isomères ortho et para des sels alcalins de l'acide hydroxymandélique par extraction sélective des mélanges aqueux. En faisant une extraction du mélange avec un solvant organique aprotique polaire, par exemple l'acétone, la méthylcétone, le tétrahydrofuranne, l'acétate d'éthyle, il a été trouvé que le sel de sodium de l'isomère ortho était très soluble dans ces solvants. Mais un tel procédé exige de nombreuses extractions et évaporations et l'utilisation répétée de solvants, ce qui rend le procédé difficilement industrialisable, coûteux et source de pollution. De plus, aucun des produits obtenus n'a la pureté exigée dans certaines applications comme par exemple la synthèse de principes actifs pour l'industrie pharmaceutique ou phytopharmaceutique.

FR-A-2.687.143 précédemment cité décrit un mode de préparation discontinu permettant d'obtenir l'acide orthohydroxymandélique avec un rendement pouvant aller jusqu'à 86,5 % par condensation de l'acide glyoxylique sur le phénol en présence d'une amine tertiaire, de préférence la tributylamine et de quantités catalytiques de cations métalliques trivalents, de préférence Al$_2$(SO$_4$)$_3$, les produits secondaires de cette réaction étant l'acide parahydroxymandélique et un produit disubstitué en quantité très faible. Mais il restait à isoler l'acide orthohydroxymandélique ou ses sels alcalins à l'état pur.

Pour préparer l'orthohydroxymandélate de sodium en continu dans une installation industrielle selon le principe général décrit dans FR-A-2.687.143, il était nécessaire de déterminer, puis de maîtriser les paramètres essentiels de cette condensation, de manière à réduire les réactions secondaires précédemment citées pour obtenir le meilleur rendement possible à partir de l'acide glyoxylique mis en oeuvre. Il était aussi indispensable que ce procédé permette un recyclage maximal de toutes les matières premières mises en oeuvre et non transformées. Il est également nécessaire que le procédé présente une productivité horaire satisfaisante pour rentabiliser les investissements réalisés. Une fiabilité des installations était requise et le procédé devait fournir une sélectivité correcte de la condensation pour minimiser les quantités de produits secondaires et en particulier le parahydroxymandélate de sodium. Le procédé devait enfin conduire aux coûts d'exploitation les plus faibles possibles.

La demanderesse a essayé de réaliser la condensation du phénol avec de l'acide glyoxylique, par réaction dans une série de réacteurs. Mais en opérant en milieu concentré, il se posait un problème important de bouchage lors du transfert du mélange réactionnel entre le premier et le second réacteur.

Après de longues études supplémentaires, ce problème a été résolu en faisant réagir du phénol avec de l'acide glyoxylique en solution aqueuse en présence d'une amine tertiaire et de quantités catalytiques de cations métalliques trivalents successivement dans au moins deux réacteurs, le mélange résultant étant traité successivement dans deux mélangeurs décanteurs, la phase solvant issue du second mélangeur décanteur étant renvoyée vers les produits de départ.

C'est pourquoi la présente invention a pour objet un procédé industriel de fabrication en continu de l'orthohydroxymandélate de sodium par condensation en atmosphère inerte du phénol avec de l'acide glyoxylique en solution aqueuse, en présence d'une amine tertiaire et de quantités catalytiques d'un cation métallique trivalent à une température inférieure à 100 °C, caractérisé par le fait que le procédé est réalisé en continu dans au moins trois réacteurs (R1 ..Rn) montés en série, le premier étant alimenté par une première cuve C1 renfermant l'acide glyoxylique et les cations métalliques trivalents, et une seconde cuve C2 renfermant le phénol et l'amine tertiaire, que le milieu réactionnel obtenu à la sortie du dernier des au moins trois réacteurs et composé d'une phase aqueuse et d'une phase organique est transféré dans le premier de deux mélangeurs décanteurs (MD1 et MD2) montés en série, que l'on ajoute dans le premier mélangeur décanteur MD1 un hydroxyde alcalin et un éther solvant du phénol et de l'amine tertiaire, que l'on récupère la phase aqueuse du premier mélangeur - décanteur MD1 pour en extraire l'orthohydroxymandélate de sodium attendu tandis que la phase organique issue de MD1 est transférée dans le second mélangeur-décanteur MD2 et lavée avec de l'eau, et que l'on transfère la phase organique du deuxième mélangeur-décanteur MD2 vers la seconde cuve (C2), et par le fait que le premier réacteur (R1) est maintenu à une température de 30 à 80°C, le second, (R2) à une température de 70 à 90°C et le dernier, (R3) à une température de 70 à 90°C.

L'acide glyoxylique de départ est de préférence utilisé en solution aqueuse, par exemple de 40 à 60 % en poids, et de préférence à environ 50% (acide glyoxylique dit industriel).

Le phénol de départ utilisé est par exemple à une concentration de 75 à 95% en poids dans l'eau, notamment à une concentration de 85 à 95% en poids dans l'eau et de préférence à une concentration de 90% environ en poids dans l'eau

Les amines tertiaires utilisées peuvent être solubles dans l'eau, comme le diméthylaminopropanol, le diméthylaminodiglycol, la tétraméthylène-diamine. On préfère les amines tertiaires insolubles dans l'eau, comme la trihexylamine, la N-tripropylamine, la triisononylamine, la diméthyl-cyclohexylamine, et de préférence, la tributylamine pour les récupérer au niveau des phases d'extraction aqueuses.

Des cations métalliques trivalents convenables comprennent les cations chrome III, fer III, aluminium III, gallium III, indium III, thallium III, ruthenium III, scandium III, mais de préférence les cations chrome III, fer III et aluminium III, et tout particulièrement ce dernier.

Un couple particulièrement avantageux d'amine tertiaire et de cation métallique trivalent est constitué de tributylamine et d'aluminium.

Dans des conditions de réalisation préférées, le procédé ci-dessus est caractérisé en ce que la phase organique issue du second mélangeur-décanteur MD2 est mélangée à une solution aqueuse de phénol et concentrée pour éliminer tout ou partie de la phase aqueuse, puis envoyée vers la seconde cuve (C2).

De manière préférentielle, le procédé est caractérisé par le fait que le temps de séjour du milieu réactionnel dans chacun des réacteurs est compris entre 30 et 120 minutes, notamment environ 45 mn.

Dans d'autres conditions préférées, l'on envoie la phase aqueuse provenant du premier mélangeur-décanteur (MD1) dans une colonne d'extraction (S) ou elle est mélangée à un éther solvant du phénol et de l'amine tertiaire, la phase organique en résultant étant envoyée vers la seconde cuve (C2) en même temps que celle issue du second mélangeur-décanteur (MD2) tandis que l'on récupère la phase aqueuse renfermant l'orthohydroxymandélate de sodium attendu.

Dans encore d'autres conditions opératoires préférées, on utilise trois réacteurs en série et la température du premier réacteur est comprise entre 30 et 80°C, celle du deuxième réacteur entre 70 et 90°C, et celle du troisième réacteur entre 70 et 90°C.

Dans des conditions tout particulièrement préférées, le procédé est réalisé dans trois réacteurs maintenus à 75 ± 5 °C pour le premier, 85 ± 5 °C pour les deuxième et troisième réacteur.

Dans toujours d'autres conditions préférées, le procédé de l'invention est aussi caractérisé par le fait que le mélange issu des 3 réacteurs en série est envoyé dans deux mélangeurs décanteurs MD1 et MD2 successifs. Ce mélange est envoyé dans MD1 en même temps qu'une solution aqueuse de soude, et la phase aqueuse 1 de ce premier mélangeur décanteur MD1 est envoyée vers une colonne d'extraction agitée, S, d'où sera extraite la solution finale de l'orthohydroxymandélate de sodium. Le deuxième mélangeur décanteur MD2 est utilisé pour laver à l'eau la phase organique sortant de MD1, avant retour dans le procédé vers la seconde cuve (C2).

Le procédé de l'invention est encore notamment caractérisé par le fait que la phase organique sortant de la colonne d'extraction S est envoyée vers un concentreur D, de préférence en même temps que la phase organique provenant de MD2.

Dans des conditions opératoires préférées, la proportion molaire des réactifs de départ est pour une mole d'acide glyoxylique :

- 1 à 15 moles de phénol,
- 0,8 à 1,2 moles d'amine tertiaire,

- 0,001 à 0,1 mole de cation métallique trivalent.

Les réactifs de départ proviennent avantageusement de 2 cuves d'alimentation, l'une, C1, contenant l'acide glyoxylique et le cation métallique trivalent, l'autre, C2, contenant l'amine tertiaire et le phénol. Ces cuves alimentent préférentiellement en continu le premier réacteur, à débit constant, avec une solution aqueuse contenant pour 1 mole d'acide glyoxylique, 1 à 15 moles de phénol, 0,8 à 1,2 moles d'amine tertiaire, 0,001 à 0,1 mole de cation métallique trivalent.

Dans encore d'autres conditions préférentielles de mise en oeuvre du procédé ci-dessus, le mélange réactionnel de R1 coule par débordement dans un réacteur R2 puis dans un réacteur R3.

A la sortie du dernier réacteur, par exemple R3 s'il y a trois réacteurs, le mélange réactionnel est envoyé dans un premier mélangeur décanteur MD1 dans lequel on ajoute notamment un hydroxyde alcalin, de préférence la soude ou la potasse, et un éther solvant du phénol et de l'amine tertiaire tel que le méthyltertiobutyléther (MTBE), le diisopropyléther (DIPE) ou de préférence le tertioamylméthyléther (TAME). On utilise avantageusement conjointement la soude avec le TAME. On obtient 2 phases distinctes : une phase aqueuse 1 et une phase solvant 1.

La phase solvant 1 passe, de préférence par débordement, dans un deuxième mélangeur décanteur MD2, où elle est lavée par de l'eau. Ce lavage a pour effet d'extraire le reste des mandélates de la phase organique.

A la sortie de MD2 on récupère à nouveau 2 phases : une phase aqueuse 2 et une phase solvant 2. La phase aqueuse 2 est envoyée vers MD1 et sert en partie à diluer la soude ; la phase solvant 2 est envoyée vers la cuve C2 par l'intermédiaire d'un concentreur D.

En vue d'isoler l'orthohydroxymandélate de sodium, la phase aqueuse 1 est de préférence envoyée vers une colonne d'extraction S. La phase organique sortant de la colonne d'extraction S est avantageusement envoyée vers un concentreur D en même temps que la phase solvant 2.

Dans le concentreur D, on élimine avantageusement l'eau et le TAME qui pourront ensuite être recyclés dans le procédé.

De façon encore plus préférentielle, le procédé est caractérisé par le fait que, la solution aqueuse de départ contient pour 1 mole d'acide glyoxylique, 12 moles de phénol, 1,05 mole de tributylamine, 0,005 mole de sulfate d'alumine. Dans d'autres conditions préférentielles de mise en oeuvre de l'invention, les réacteurs R1, R2 et R3 ont une capacité respective de 5 m$^3$, 10 m$^3$ et 10 m$^3$. Dans encore d'autres conditions préférentielles de mise en oeuvre de l'invention, les temps de contact des réactifs dans chacun des réacteurs est de 45 mn environ. Dans des conditions tout à fait préférées, les températures des réacteurs R1, R2 et R3 sont respectivement de 75, 85 et 85°C.

Dans une mise en oeuvre préférée, le mélange réactionnel issu des 3 réacteurs est envoyé dans 2 mélangeurs décanteurs en série, MD1 et MD2, de préférence de 2m$^3$. La phase aqueuse 1 du premier mélangeur MD1 est avantageusement envoyée vers une colonne d'extraction agitée S, notamment de diamètre DN 450 d'où l'on récupère en solution aqueuse l'orthohydroxymandélate de sodium désiré.

## EXEMPLE 1

a) Préparation des réactifs

Dans une cuve C1 de 250 litres, on effectue un pré-mélange d'acide glyoxylique en solution aqueuse à 50 % en poids et de sulfate d'aluminium en solution aqueuse à 37 % dans un rapport molaire

$$\frac{\text{acide glyoxylique } 1}{Al^{3+}} = \frac{1}{0,05}$$

le tout étant chauffé à 60°C.

Dans une cuve C2 de 25 m$^3$ on prépare un mélange tributylaminephénol dans un rapport molaire

$$\frac{\text{tributylamine}}{\text{phénol}} = \frac{1,05}{12}$$

La température intérieure de cette cuve C2 est réglée à 75°C, et elle est reliée à un concentreur D.

b) Etape réactionnelle dans les 3 réacteurs R1, R2 et R3

A partir des mélanges contenus dans les cuves C1 et C2, on alimente un premier réacteur R1 de 5 m3 avec un débit tel que le rapport molaire acide glyoxylique / sulfate d'alumine / tributylamine / phénol soit égal à 1 / 0,05 / 1,05 / 12 et que le temps de séjour dans ce réacteur soit de 45 mn. La température intérieure de ce réacteur est de 75°C.

Le mélange réactionnel coule par débordement dans un réacteur R2 de 10 m$^3$ et y est maintenu 45 mn à 85°C, puis dans un réacteur R3 de 10 m$^3$ où il y est maintenu 45 mn à 85°C.

c) Etapes de séparation des produits réactionnels

A la sortie de ce réacteur R3 le mélange réactionnel appelé MR, est envoyé vers un mélangeur - décanteur MD1 de 2 m$^3$ qui est maintenu à une température de 54°C.

On ajoute de façon continue dans MD1 de la soude diluée à 17%, préparée à partir de soude à 47 % et d'eau dont une partie provient de la décantation de l'azéotrope obtenu dans le décanteur D et une partie provient du mélangeur - décanteur MD2, de façon à respecter un rapport molaire

$$\frac{\text{Soude}}{\text{acide glyoxylique}} = 1{,}8$$

On ajoute également dans MD1 du tertioamylméthyléther (TAME) de façon à respecter un rapport molaire

$$\frac{\text{TAME}}{\text{milieu réactionnel}} = 0{,}5$$

A la sortie de MD1, on soutire 2 phases distinctes : une phase aqueuse 1 et une phase solvant 1.

La phase solvant 1 passe par débordement dans un deuxième mélangeur - décanteur (MD2) de 2 m3 où elle est lavée par de l'eau, de telle façon que le rapport volumique

$$\frac{\text{eau}}{\text{phase solvant 1}} = 0{,}2$$

A la sortie de MD2 on récupère à nouveau 2 phases : une phase aqueuse 2 et une phase solvant 2. La phase aqueuse 2 est envoyée vers MD1 et sert en partie à diluer la soude, la phase solvant 2 est envoyée vers un concentreur D.

La phase aqueuse 1 est envoyée vers une colonne d'extraction S agitée, de diamètre DN 450, qui permet de récupérer l'orthohydroxymandélate de sodium désiré.

Dans cette colonne d'extraction, on réalise à 35 - 40°C l'extraction du phénol solubilisé en phase aqueuse par du TAME

$$(\text{rapport volumique} \quad \frac{\text{phase aqueuse 1}}{\text{TAME}} = 1{,}8)$$

La phase organique sortant de cette colonne d'extraction S est envoyée vers le concentreur D en même temps que la phase solvant 2 et du phénol à 90 % avec un débit tel que les rapport molaires

$$\frac{\text{phénol 90 \%}}{\text{phénol phase solvant entrant}} = \frac{1}{11}$$

Dans le concentreur D, on élimine l'eau et le TAME qui seront recyclés ensuite dans le procédé. On obtient après concentration une solution

$$\frac{\text{tributylamine}}{\text{phénol}} \quad \text{dans le rapport molaire} \quad \frac{\text{TBA}}{\text{phénol}} = \frac{1{,}05}{12}$$

Cette dernière solution est recyclée via la cuve de réactifs C2 dans le procédé.

La phase aqueuse sortant de colonne d'extraction S est la solution finale d'orthohydroxymandélate de sodium qui après élimination du reste du solvant par la vapeur, pourra servir de matière première.

Des échantillons ont été prélevés dans les réacteurs R1, R2 et R3, dans les phases solvant 1 et aqueuse 1 de MD1, dans les phases solvant 2 et aqueuse 2 de MD2, et la solution aqueuse finale d'orthohydroxymandélate de sodium, ont été analysés par chromatographie liquide haute pression (HPLC).

On a également déterminé la sélectivité de la réaction calculée selon l'équation 1 :

$$S= \frac{100 \text{ x mole OHMNa}}{\text{mole OHMNa + mole PHMNa + 2 moles diglycolate Na}}$$

et le rendement de la réaction selon l'équation 2 :

$$R = \frac{(\text{mole OHMNa + mole PHMNa + 2 moles diglycolates Na })}{\text{mole acide glyoxylique libre ou salifié}} \text{ x } 100$$

Les résultats mentionnés dans le tableau suivant sont exprimés en pourcentages molaires :

| | OHMNa | PHMNa | Diglycolates | Sélectivité | Rendement |
|---|---|---|---|---|---|
| Réacteur R1 | 0,25 | 0,025 | - | 90,9 | 27,5 |
| Réacteur R2 | 0,69 | 0,11 | 0,005 | 85,2 | 81 |
| Réacteur R3 | 0,81 | 0,13 | 0,01 | 84,4 | 96 |
| Phase solvant 1 | 0,04 | 0,01 | - | | |
| Phase aqueuse 1 | 0,77 | 0,13 | 0,01 | | |
| Phase solvant 2 | 0,05 | - | - | | |
| Phase aqueuse 2 | 0,035 | - | - | | |
| Solution aqueuse OHMNa | 0,77 | 0,13 | 0,01 | 83,7 | 92 |

On constate que la sélectivité et le rendement de la synthèse en continu sont en tous points comparables à ceux décrits dans FR-A-2.687.143

## Revendications

1. Procédé industriel de fabrication en continu de l'orthohydroxymandélate de sodium par condensation en atmosphère inerte du phénol avec de l'acide glyoxylique en solution aqueuse, en présence d'une amine tertiaire et de quantités catalytiques d'un cation métallique trivalent à une température inférieure à 100 °C, **caractérisé par le fait que** le procédé est réalisé en continu dans au moins trois réacteurs (R1 ... Rn) montés en série, le premier étant alimenté par une première cuve C1 renfermant l'acide glyoxylique et les cations métalliques trivalents, et une seconde cuve C2 renfermant le phénol et l'amine tertiaire, que le milieu réactionnel obtenu à la sortie du dernier des au moins trois réacteurs et composé d'une phase aqueuse et d'une phase organique est transféré dans le premier de deux mélangeurs décanteurs (MD1 et MD2) montés en série, que l'on ajoute dans le premier mélangeur décanteur MD1 un hydroxyde alcalin et un éther solvant du phénol et de l'amine tertiaire, que l'on récupère la phase aqueuse du premier mélangeur - décanteur MD1 pour en extraire l'orthohydroxymandélate de sodium attendu tandis que la phase organique issue de MD1 est transférée dans le second mélangeur-décanteur MD2 et lavée avec de l'eau, et que l'on transfère la phase organique du deuxième mélangeur-décanteur MD2 vers la seconde cuve (C2), et **par le fait que** le premier réacteur (R1) est maintenu à une température de 30 à 80°C, le second, (R2) à une température de 70 à 90°C et le dernier, (R3) à une température de 70 à 90°C.

2. Procédé selon la revendication 1, **caractérisé par le fait que** la phase organique issue du second mélangeur-décanteur MD2 est mélangée à une solution aqueuse de phénol et concentrée pour éliminer tout ou partie de la phase aqueuse, puis envoyée vers la seconde cuve (C2).

3. Procédé selon la revendication 2, **caractérisé par le fait que** l'on envoie la phase aqueuse provenant du premier mélangeur-décanteur (MD1) dans une colonne d'extraction (S) ou elle est mélangée à un éther solvant du phénol et de l'amine tertiaire, la phase organique en résultant étant envoyée vers la seconde cuve (C2) en même temps que celle issue du second mélangeur-décanteur (MD2) tandis que l'on récupère la phase aqueuse renfermant l'orthohydroxymandélate de sodium attendu.

4. Procédé selon la revendication 1, **caractérisé par le fait que** la proportion molaire des réactifs de départ est 1 mole d'acide glyoxylique pour 1 à 15 moles de phénol, pour 0,8 à 1,2 moles d'amine tertiaire, et pour 0,001 à 0,1 mole de cation métallique trivalent.

**5.** Procédé selon l'une des revendications 1 à 4, **caractérisé par le fait que** l'amine tertiaire est la tributylamine, et que le cation métallique trivalent est l'aluminium.

**6.** Procédé selon l'une des revendications 1 à 5, **caractérisé par le fait que** les temps de séjour du milieu réactionnel dans chaque réacteur sont tels qu'ils soient compris chacun entre 30 et 120 minutes.

**7.** Procédé selon la revendication 6, **caractérisé par le fait que** le temps de séjour du milieu réactionnel dans chaque réacteur est d'environ 45 minutes.

**8.** Procédé selon l'une des revendications 1 à 7, **caractérisé par le fait que** le procédé est réalisé dans trois réacteurs maintenus à $75 \pm 5 \, °C$ pour le premier, $85 \pm 5 \, °C$ pour les deuxième et troisième réacteur.

**9.** Procédé selon l'une des revendications 1 à 8, **caractérisé par le fait que** l'on ajoute dans le premier mélangeur-décanteur MD1, de la soude ou de la potasse, et un éther solvant du phénol et de l'amine tertiaire.

**10.** Procédé selon la revendication 9, **caractérisé par le fait que** l'hydroxyde alcalin est la soude, et que l'éther est le tertioamylméthyléther (TAME).

**11.** Procédé selon l'une des revendications 3 et 5 à 10, **caractérisé par le fait que** la phase aqueuse sortant de la colonne d'extraction S est la solution finale de l'orthohydroxymandélate de sodium recherchée.

**12.** Procédé selon la revendication 1, **caractérisé par le fait que** le premier réacteur est alimenté en continu, à débit constant, avec une solution aqueuse contenant pour 1 mole d'acide glyoxylique, 1 à 15 moles de phénol, 0,8 à 1,2 moles d'amine tertiaire, 0,001 à 0,1 mole de cation métallique trivalent.

**Patentansprüche**

**1.** Kontinuierliches industrielles Herstellungsverfahren für Natriumorthohydroxymandelat durch Kondensation von Phenol mit Glyoxylsäure in wässeriger Lösung in Gegenwart eines tertiären Amins und katalytischer Mengen eines dreiwertigen Metallkations bei einer Temperatur unterhalb 100°C in inerter Atmosphäre, **dadurch gekennzeichnet, daß** das Verfahren kontinuierlich in mindestens drei in Serie geschalteten Reaktoren (R1...Rn) durchgeführt wird, wobei der erste durch eine erste Wanne C1 versorgt wird, die Glyoxylsäure und die dreiwertigen Metallkationen enthält, und eine zweite Wanne C2, die das Phenol und das tertiäre Amin enthält, daß das am Austritt des letzten der mindestens drei Reaktoren erhaltene und das aus einer wässerigen Phase und einer organischen Phase zusammengesetzte Reaktionsmedium in den ersten von zwei in Reihe geschalteten Mischungsdekantern (MD1 und MD2) überführt wird, daß man in den ersten Mischungsdekanter MD1 ein alkalisches Hydroxid und ein Etherlösungsmittel für das Phenol und das tertiäre Amin gibt, daß man die wässerige Phase des ersten Mischungsdekanters MD1 wiedergewinnt, um das erwartete Natriumorthohydroxymandelat zu extrahieren, während die von MD1 stammende organische Phase in den zweiten Mischungsdekanter MD2 überführt und mit Wasser gewaschen wird, und daß man die organische Phase des zweiten Mischungsdekanters MD2 in die zweite Wanne (C2) überführt und daß der erste Reaktor (R1) bei einer Temperatur von 30 bis 80°C und der zweite (R2) bei einer Temperatur von 70 bis 90°C und der letze (R3) bei einer Temperatur von 70 bis 90 °C gehalten wird.

**2.** Verfahren nach Anspruch 1, **dadurch gekennzeichnet, daß** die aus dem zweiten Mischungsdekanter MD2 stammende organische Phase mit einer wässerigen Phenollösung gemischt und konzentriert wird, um die wässerige Phase ganz oder teilweise zu entfernen und dann zur zweiten Wanne (C2) zu führen.

**3.** Verfahren nach Anspruch 2, **dadurch gekennzeichnet, daß** man die aus dem ersten Mischungsdekanter (MD1) stammende wässerige Phase in eine Exraktionssäule (S) führt, wo diese mit einem Etherlösungsmittel für das Phenol und das tertiäre Amin gemischt wird, die daraus resultierende organische Phase zur zweiten Wanne (C2) geführt wird, sowie sie im zweiten Mischungsdekanter (MD2) entstanden ist, während man die das erwartete Natriumorthohydroxymandelat enthaltende wässerige Phase wiedergewinnt.

**4.** Verfahren nach Anspruch 1, **dadurch gekennzeichnet, daß** die molare Menge der Ausgangsreagenzien 1 Mol Glyoxylsäure für 1 bis 15 Mol Phenol, 0,8 bis 1,2 Mol tertiäres Amin und 0,001 bis 0,1 Mol dreiwertiges Metallkation beträgt.

**5.** Verfahren nach einem der Ansprüche 1 bis 4, **dadurch gekennzeichnet, daß** das tertiäre Amin Tributylamin ist und daß das dreiwertige metallische Kation Aluminium darstellt.

**6.** Verfahren nach einem der Ansprüche 1 bis 5, **dadurch gekennzeichnet, daß** die Verweilzeiten des Reaktionsmediums in jedem Reaktor so sind, daß sie jeweils zwischen 30 und 120 min umfassen.

**7.** Verfahren nach Anspruch 6, **dadurch gekennzeichnet, daß** die Verweilzeit des Reaktionsmediums in jedem Reaktor etwa 45 min beträgt.

**8.** Verfahren nach einem der Ansprüche 1 bis 7, **dadurch gekennzeichnet, daß** das Verfahren in drei Reaktoren durchgeführt wird, die bei 75 ± 5°C für den ersten, 85 ± 5°C für den zweiten und dritten Reaktor gehalten werden.

**9.** Verfahren nach einem der Ansprüche 1 bis 8, **dadurch gekennzeichnet, daß** man im ersten Mischungsdekanter MD1 Natronlauge oder Kalilauge und ein Etherlösungsmittel für Phenol und das tertiäre Amin zugibt.

**10.** Verfahren nach Anspruch 9, **dadurch gekennzeichnet, daß** das alkalische Hydroxid Natronlauge ist, und der Ether tert-Amylmethylether (TAME) darstellt.

**11.** Verfahren nach einem der Ansprüche 3 und 5 bis 10, **dadurch gekennzeichnet, daß** die wässerige Phase, die die Extraktionssäule S verlässt, die endgültige Lösung des gesuchten Natriumorthohydroxymandelats darstellt.

**12.** Verfahren nach Anspruch 1, **dadurch gekennzeichnet, daß** der erste Reaktor kontinuierlich mit konstantem Duchsatz mit einer wässerigen Lösung versorgt wird, enthaltend für 1 Mol Glyoxylsäure 1 bis 15 Mol Phenol, 0,8 bis 1,2 Mol tertiäres Amin und 0,001 bis 0,1 Mol dreiwertiges metallisches Kation.

## Claims

**1.** Industrial process for the continuous manufacture of sodium orthohydroxymandelate by condensation of phenol in an inert atmosphere with glyoxylic acid in aqueous solution, in the presence of a tertiary amine and of catalytic quantities of a trivalent metal cation at a temperature below 100°C, **characterized by** the fact that the process is carried out continuously in at least three reactors (R1 ... Rn) installed in series, the first being supplied by a first tank C1 containing the glyoxylic acid and the trivalent metal cations, and by a second tank C2 containing the phenol and the tertiary amine, that the reaction medium obtained at the outlet of the last of the at least three reactors and consisting of an aqueous phase and an organic phase is transferred into the first of two mixer-decanters (MD1 and MD2) installed in series, that an alkaline hydroxide and an ether which dissolves phenol and the tertiary amine are added to the first mixer-decanter MD1, that the aqueous phase of the first mixer-decanter MD1 is recovered in order to extract the expected sodium orthohydroxymandelate from it, while the organic phase that has come from MD1 is transferred into the second mixer-decanter MD2 and washed with water, and that the organic phase of the second mixer-decanter MD2 is transferred to the second tank (C2), and by the fact that the first reactor (R1) is kept at a temperature of 30 to 80°C, the second (R2) at a temperature of 70 to 90°C and the last (R3) at a temperature of 70 to 90°C.

**2.** Process according to claim 1, **characterized by** the fact that the organic phase that has come from the second mixer-decanter MD2 is mixed with an aqueous solution of phenol and concentrated in order to eliminate all or part of the aqueous phase, then sent to the second tank (C2).

**3.** Process according to claim 2, **characterized by** the fact that the aqueous phase coming from the first mixer-decanter (MD1) is sent into an extraction column (S) where it is mixed with an ether which dissolves the phenol and the tertiary amine, the organic phase resulting from this being sent to the second tank (C2) at the same time as that which has come from the second mixer-decanter (MD2), while the aqueous phase containing the expected sodium orthohydroxymandelate is recovered.

**4.** Process according to claim 1, **characterized by** the fact that the molar proportion of the starting reagents is 1 mole of glyoxylic acid for 1 to 15 moles of phenol, for 0.8 to 1.2 moles of tertiary amine, and for 0.001 to 0.1 mole of trivalent metal cation.

**5.** Process according to one of claims 1 to 4, **characterized by** the fact that the tertiary amine is tributylamine, and the trivalent metal cation is aluminium.

6. Process according to one of claims 1 to 5, **characterized by** the fact that the residence times of the reaction medium in each reactor are such that each is between 30 and 120 minutes.

7. Process according to claim 6, **characterized by** the fact that the residence time of the reaction medium in each reactor is about 45 minutes.

8. Process according to one of claims 1 to 7, **characterized by** the fact that the process is carried out in three reactors kept at 75 ± 5°C for the first, 85 ± 5°C for the second and third reactors.

9. Process according to one of claims 1 to 8, **characterized by** the fact that soda or potash, and an ether which dissolves the phenol and the tertiary amine, are added to the first mixer-decanter MD1.

10. Process according to claim 9, **characterized by** the fact that the alkaline hydroxide is soda, and the ether is tert-amyl methyl ether (TAME).

11. Process according to one of claims 3 and 5 to 10, **characterized by** the fact that the aqueous phase leaving the extraction column S is the final sought sodium orthohydroxymandelate solution.

12. Process according to claim 1, **characterized by** the fact that the first reactor is supplied continuously, at a constant rate of feed, with an aqueous solution containing, for 1 mole of glyoxylic acid, 1 to 15 moles of phenol, 0.8 to 1.2 moles of tertiary amine, and 0.001 to 0.1 mole of trivalent metal cation.